# FASCICULE DE BREVET EUROPEEN

(11) **EP 0 723 934 B1**
(45) Date de publication et mention de la délivrance du brevet: **28.07.1999**
(21) Numéro de dépôt: 96420025.7
(22) Date de dépôt: 22.01.1996
(51) Int. Cl.: C01G 31/02, C07C 253/26, B01J 23/22

(54) **Catalyseurs d'ammoxydation et leur procédé de préparation**
Ammoxidationskatalysatoren und Verfahren zu ihrer Herstellung
Ammoxidation catalysts and their process of preparation

(30) Priorité: 24.01.1995 FR 9500999
(43) Date de publication de la demande: 31.07.1996
(73) Titulaire: RHODIA FIBER & RESIN INTERMEDIATES, 92408 Courbevoie Cédex (FR)
(72) Inventeur: Albonetti, Stéfania, I-40026 Imola (IT); Blanchard, Gilbert, F-60330 Le Plessis Belleville (FR); Burattin, Paolo, F-75013 Paris (FR); Cavani, Fabrizio, I-41100 Modena (IT); Trifiro, Ferruccio, I-40136 Bologna (IT)
(74) Mandataire: Vignally, Noel

(56) Documents cités:
- EP-A- 0 003 158
- EP-A- 0 492 805
- WO-A-92/01631
- US-A- 5 258 543

## Description

La présente invention concerne des oxydes mixtes de vanadium, d'antimoine et de titane, comportant une phase cristalline de type TiO₂ rutile.

Elle concerne plus particulièrement un procédé de préparation de ces oxydes mixtes, ainsi que leur utilisation comme catalyseurs pour l'ammoxydation des alcanes. Certains oxydes mixtes de vanadium et d'antimoine ou de vanadium, d'antimoine et d'autres métaux sont des composés connus qui ont été décrits, parmi de nombreux autres oxydes mixtes, dans le brevet FR-A-2 072 334.

De même, dans le brevet EP-A-0 492 805, il a été décrit la préparation d'innombrables composés, parmi lesquels des oxydes mixtes de vanadium, d'antimoine, d'étain et de titane, par le mélange en solution aqueuse de peroxyde d'hydrogène de composés de divers métaux, suivi par la calcination dudit mélange à une température supérieure à 750°C, de préférence supérieure à 780°C, puis le traitement du mélange calciné par un composé monohydroxylé ou dihydroxylé. Tous ces composés présentent un rapport molaire Ti/V de 0,1 ou 0,2.

La présente invention concerne tout d'abord un procédé de préparation d'un oxyde mixte comportant du vanadium, de l'antimoine et du titane, répondant à la formule empirique générale (I):

V Sbₐ Ti_{b} Oₓ (I)

dans laquelle :
- a représente un nombre entier ou fractionnaire égal ou supérieur à 0,1
- b représente un nombre entier ou fractionnaire égal ou supérieur à 0,1
- x représente un nombre entier ou fractionnaire déterminé par le degré d'oxydation des autres éléments,
   caractérisé en ce que :
- des composés respectifs de vanadium, d'antimoine et de titane sont mis en solution dans au moins un alcool saturé,
- la solution alcoolique ainsi obtenue est mise en contact avec de l'eau, afin de précipiter l'oxyde mixte,
- l'oxyde mixte obtenu est séparé et calciné.

Ces oxydes mixtes présentent les raies de diffraction X caractéristiques d'une phase cristallographique de type TiO₂ rutile et ne présentant pas de raies de diffraction X caractéristiques des phases cristallographiques de α-Sb₂O₄ cervantite et/ou de Sb₆O₁₃.

Les raies caractéristiques de la phase de type TiO₂ rutile correspond aux distances inter-réticulaires d (en Å) suivantes :
3,24-3,27
2,48-2,52
2,29-2,31
2,18-2,21
2,05-2,07
1,68-1,70
1,62-1,64
1,48-1,51
1,45-1,50
1,42-1,46
1,36-1,37
1,30-1,31
1,24-1,26.

Les raies caractéristiques de la phase α-Sb₂O₄ cervantite correspondent aux distances inter-réticulaires d (en Å) suivantes :
4,45
3,45
3,07
2,93
2,73
2,65
2,47
2,40
2,24
2,16
2,00
1,91
1,86
1,78
1,72
1,63
1,59
1,56
1,52
1,47
1,42
1,37.

Les raies caractéristiques de la phase Sb₆O₁₃ correspondent aux distances inter-réticulaires d (en Å) suivantes :
5,95
3,11
2,97
2,58
2,36
2,10
1,98
1,82
1,74
1,57
1,55
1,48
1,44.

Parmi les oxydes mixtes de formule (I) définis précédemment, qui sont préparés par le procédé de l'invention, on préfère ceux pour lesquels :
- a représente un nombre entier ou fractionnaire égal ou inférieur à 100
- b représente un nombre entier ou fractionnaire égal ou inférieur à 100
- x représente un nombre entier ou fractionnaire déterminé par le degré d'oxydation des autres éléments.

Enfin on préfère encore plus particulièrement les oxydes mixtes de formule (I) pour lesquels :
- a représente un nombre entier ou fractionnaire de 0,5 à 50
- b représente un nombre entier ou fractionnaire de 1 à 40
- x représente un nombre entier ou fractionnaire déterminé par le degré d'oxydation des autres éléments.

Les composés du vanadium, de l'antimoine et du titane mis en oeuvre dans le procédé doivent être solubles dans un alcool saturé ou un mélange d'alcools saturés. Dans le présent texte, on considère qu'un composé est soluble lorsque sa solubilité mesurée à 25 °C est d'au moins 5 grammes par litre d'alcool saturé. Ces composés peuvent être introduits ensemble ; ils peuvent aussi tout d'abord être mis séparément en solution dans un alcool, les différentes solutions alcooliques ainsi obtenues étant ensuite mélangées entre elles. Généralement, mais non limitativement, on prépare une solution alcoolique par dissolution des différents composés, sans préparation intermédiaire de solutions de chacune des composés du vanadium, de l'antimoine et du titane.

A titre d'exemples de composés solubles du vanadium, on peut citer l'acétylacétonate de vanadyle, le trichlorure de vanadyle, le trifluorure de vanadium, le tétrafluorure de vanadium, le pentafluorure de vanadium, le tribromure de vanadium, le dichlorure de vanadium, le trichlorure de vanadium, le tétrachlorure de vanadium, le triiodure de vanadium.

A titre d'exemples de composés solubles de l'antimoine, on peut citer le pentachlorure d'antimoine, le trichlorure d'antimoine, le tribromure d'antimoine, le trifluorure d'antimoine, le triiodure d'antimoine, le trioxyde d'antimoine, l'hydrure d'antimoine.

A titre d'exemples de composés solubles du titane, on peut citer le dichlorure de titane, le tétrachlorure de titane, le trichlorure de titane, le tribromure de titane, le tétrabromure de titane, le tétrafluorure de titane, le diiodure de titane.

Les alcools saturés mis en oeuvre dans le procédé de l'invention sont plus particulièrement les alcanols et les cycloalcanols. De préférence, on utilisera les alcanols et cycloalcanols dont la température d'ébullition ne soit pas trop élevée, afin de faciliter les opérations de séparation ou de recyclage par distillation ou évaporation. Ainsi les alcanols ayant de 1 à 6 atomes de carbone, tels que le méthanol, l'éthanol, le n.propanol, le propanol-2, le n.butanol, le butanol-2, le tertiobutanol, les pentanols et les hexanols, et le cyclohexanol sont préférés.

La solution alcoolique obtenue précédemment est ensuite mélangée à de l'eau, de manière à précipiter les oxydes mixtes. De préférence, on opère dans une solution aqueuse d'un sel d'ammonium, notamment un carboxylate d'ammonium (par exemple acétate, citrate, tartrate), l'oxalate d'ammonium, le carbonate d'ammonium, l'hydrogénophosphate d'ammonium, permettant d'avoir un pH compris entre 5 et 9 et de préférence proche de 7. Ainsi l'acétate d'ammonium à une concentration de deux moles par litre dans l'eau présente un pH proche de 7.

Afin de maintenir la valeur du pH de la solution de préférence à une valeur proche de 7, il peut être nécessaire de neutraliser progressivement l'acidité éventuellement formée lors de la précipitation des oxydes mixtes (par exemple d'acide halohydrique formé lorsque l'on met en oeuvre un halogénure d'antimoine et/ou un halogénure de titane et/ou un halogénure de vanadium) à l'aide d'un composé basique. Il est préféré dans le procédé de l'invention de réaliser cette neutralisation par ajout progressif contrôlé d'ammoniaque.

Après précipitation des oxydes mixtes de l'invention, on les sépare du liquide alcool saturé/eau par toute technique habituellement utilisée pour ce type d'opération, notamment par filtration. On procède ensuite à un séchage des oxydes mixtes isolés, sous pression atmosphérique ou sous pression réduite, à une température située par exemple entre 30 °C et 200 °C, sans que ces valeurs aient une importance critique.

Les oxydes mixtes de formule (I) sont ensuite soumis à une calcination à une température de 400 °C à 800 °C. De préférence la calcination est effectuée à une température de 500 °C à 750 °C.

La présente invention a également pour objet un procédé d'ammoxydation d'alcanes en phase vapeur en présence d'un catalyseur solide comportant au moins une phase active comprenant au moins un oxyde mixte répondant à la formule générale empirique (I), caractérisé en ce que ledit oxyde mixte est préparé selon le procédé décrit précédemment.

Selon la présente invention des alcanes ayant de 3 à 12 atomes de carbone par molécule sont mis à réagir en phase vapeur avec de l'ammoniac et de l'oxygène en présence d'un catalyseur dont la phase active vient d'être précisée.

Bien entendu, dans le cadre du présent procédé, on peut utiliser des gaz diluants, inertes dans les conditions réactionnelles tels que l'hélium, l'azote et l'argon. De même, de la vapeur d'eau peut être ajoutée au mélange gazeux réactionnel dans de larges limites. Ainsi le gaz réactif (alcane, ammoniac, oxygène) peut être dilué par un diluant inerte et/ou par de la vapeur d'eau. Dans cet ensemble la teneur en vapeur d'eau peut varier dans de larges limites en particulier de 0 à 50 % et, de préférence entre 3 et 30 %. Pour une bonne mise en oeuvre du procédé selon l'invention, la teneur en gaz réactif sera d'au moins 3 % et de préférence d'au moins 20 %.

Au sein du gaz réactif les teneurs respectives en volume en alcane, ammoniac et oxygène peuvent varier dans de larges limites.

La teneur en alcane dans le gaz réactif est de préférence comprise entre 5 et 70 %. Celle en ammoniac est de préférence comprise entre 3 et 50 % et celle en oxygène est de préférence comprise entre 3 et 45 %.

Pour une bonne mise en oeuvre du procédé selon l'invention, la composition du mélange réactif sera de préférence choisie en dehors du domaine d'explosivité.

Au départ de propane, on obtiendra un mélange renfermant essentiellement du propylène et de l'acrylonitrile. L'acrylonitrile est un intermédiaire industriellement produit sur une large échelle, le propylène est une matière première traditionnellement utilisée pour produire de l'acrylonitrile et divers autres produits intermédiaires bien connus de l'homme de l'art.

Au départ d'isobutane, on obtiendra un mélange renfermant du méthacrylonitrile et de l'iso-butène ou des n-butènes.

Le procédé selon l'invention convient plus particulièrement à l'ammoxydation du propane.

Si l'alcane mis en oeuvre peut être de qualité technique, il ne renfermera pas de quantités importantes de composés à insaturation éthylénique. Ainsi le propane engagé ne renfermera généralement que des quantités mineures de propylène, par exemple inférieures à 10 %.

Le procédé selon l'invention est réalisé sous forme de réaction en phase vapeur. En conséquence, n'importe quel dispositif convenant à la réalisation des réactions d'ammoxydation ou d'oxydation en phase vapeur peut être utilisé. Le procédé peut être conduit en continu ou en discontinu et il peut comprendre l'utilisation d'un lit fixe ou d'un lit fluidisé.

La température de réaction est en général comprise entre 300°C et 550°C et, de préférence, entre 400°C et 500°C.

La pression totale du mélange réactionnel peut être supérieure ou égale à la pression atmosphérique. Elle est généralement comprise entre 1 et 6 bar et, de préférence entre 1 et 4 bar.

Le débit gazeux est fixé de telle sorte que la vitesse volumique horaire soit comprise entre 100 et 36000 h⁻¹ et, de préférence entre 200 et 20000 h⁻¹.

La vitesse volumique horaire se définit comme le rapport volume gazeux total/volume du catalyseur/heure.

Bien entendu, l'homme de l'art sera à même de trouver un compromis entre la température, le débit gazeux, la nature précise du catalyseur mise en oeuvre et les divers autres paramètres de la réaction compte-tenu de ses objectifs de production.

Le catalyseur utilisé dans le procédé d'ammoxydation des alcanes peut comprendre uniquement un ou plusieurs oxydes mixtes de vanadium, d'antimoine et de titane définis précédemment, constituant la phase active du catalyseur, ou comprendre en outre un oxyde minéral, tel que par exemple l'alumine, la silice, la silice-alumine, la zircone, la cérine, la magnésie, l'oxyde de titane, l'oxyde de niobium, sur lequel ladite phase active est déposée ou avec lequel la phase active est mélangée, en utilisant différentes techniques connues de l'homme du métier, telles que l'imprégnation ou le dépôt par "slurry".

La phase catalytique, constituée de la phase active seule ou de la phase active déposée sur un oxyde minéral ou mélangée avec ledit oxyde minéral, peut ensuite être mise en oeuvre sous forme massique ou à l'état particulaire ; elle peut donc être utilisée sous forme de poudre ou être mise en forme, par exemple de billes, pastilles, extrudés, particules concassées , selon différentes techniques connues.

Pour une mise en oeuvre du procédé en lit fixe, ces techniques peuvent être, par exemple, le pastillage, l'enrobage sur un substrat inerte ou sur substrat céramique ou métallique de type monolithique.

Pour une mise en oeuvre du procédé en lit mobile ou en lit fluidisé, la phase catalytique est généralement mise en forme par atomisation, séchage et calcination.

Les phases catalytiques peuvent par exemple être mises en forme par compression, de façon à obtenir des pastilles. Ces pastilles peuvent ensuite être éventuellement concassées en éclats. Les valeurs précises de la pression, du diamètre et de l'épaisseur des pastilles, et de la granulométrie des éclats pourront être choisies par l'homme de l'art en fonction de la perte de charge admissible dans le réacteur.

Une variante de préparation de la phase catalytique peut consister à réaliser en une seule étape, la synthèse de la phase active et son dépôt sur un oxyde minéral ou son mélange avec ledit oxyde minéral.

Les phases catalytiques peuvent également être déposées sur un substrat inerte ou l'enrober. La nature de ce substrat n'est pas critique dès lors qu'il est chimiquement inerte vis-à-vis des réactifs dans les conditions réactionnelles choisies. A titre d'exemples de substrats susceptibles de convenir à la préparation de catalyseurs utilisables dans le cadre du procédé selon l'invention, on peut citer : la silice, l'alumine, la silice-alumine, l'argile frittée, le carborandum, la magnésie, le silicate de magnésium, la terre de diatomée. Ce substrat est de préférence non poreux et peut notamment être à base d'oxyde réfractaire sous forme particulaire, le substrat le plus couramment employé étant à base d'argile. Ce substrat peut par exemple être constitué de billes d'argile, inertes, pleines, solides et rugueuses, de diamètre compris entre 0.5 et 6 mm.

La valeur précise du diamètre des billes pourra être choisie en fonction de la perte de charge admissible dans le réacteur. Ce substrat peut également être rendu non poreux par émaillage.

Ce substrat peut également être un substrat céramique, ledit substrat étant de préférence sous forme d'une structure inerte et rigide de type monolithique comprenant des canaux ou conduits. De tels supports sont bien connus et ont été largement décrits dans la littérature. Les substrats en matières céramiques utilisés sont notamment ceux comprenant comme matière principale la cordiérite, l'alumine, la mullite, la porcelaine, les carbures de bore ou de silicium.

Ce substrat peut également être un substrat métallique. De tels substrats sont bien connus. Les substrats métalliques convenables sont notamment ceux obtenus à partir d'alliages de fer, de nickel et de chrome, ou ceux obtenus à partir d'alliages de fer, de chrome, aluminium et cobalt tels que ceux connus sous la marque KANTHAL ou ceux obtenus à partir d'alliages de fer, de chrome, d'aluminium et d'yttrium connus sous la marque FECRALLOY. Le métal peut aussi être de l'acier carboné ou de la fonte simple.

Lorsqu'on fait appel à un catalyseur enrobé, la quantité de phase catalytique qui peut varier dans de larges limites, est en pratique comprise entre 1 et 50 % et de préférence entre 5 et 35 % en poids par rapport à l'ensemble substrat + phase catalytique.

Ainsi, certains catalyseurs, utiles pour une mise en oeuvre du procédé en lit fixe, peuvent être obtenus par enrobage de manière connue en soi, des phases catalytiques, intermédiaires ou finies, broyées. Cette méthode classique consiste à déposer autour de billes inertes, mais rugueuses, une couche de phase catalytique intermédiaire ou finie. Une fois les billes recouvertes de la quantité voulue de la phase catalytique, elles sont séchées par de l'air chaud, entre 70 et 150°C pendant au moins 30 minutes, puis introduites dans un four pour être calcinées entre 300 et 600°C de préférence entre 450 et 500°C, pendant au moins 3 heures.

Certains catalyseurs utiles pour une mise en oeuvre du procédé selon l'invention en lit mobile ou en lit fluidisé peuvent être obtenus par la technique connue en soi du séchage par atomisation en atmosphère, de préférence non réductrice. Par une telle opération, le cas échéant suivie d'une calcination à une température de l'ordre de 400 à 800°C, on obtient des poudres de forme sphérique de diamètre compris entre 5 et 700 µm. Des poudres constituées pour au moins 80 % en poids de particules dont la dimension est comprise entre 5 et 200 µm sont préférées dans le cadre d'une utilisation en lit fluidisé.

La phase catalytique, seule ou ainsi mise en oeuvre sous forme massique ou à l'état particulaire, constitue le catalyseur.

Les produits de la réaction peuvent être récupérés dans les gaz effluents par tout moyen approprié. Par exemple les gaz effluents peuvent passer dans un condenseur contenant de l'acide sulfurique dilué pour neutraliser l'ammoniac non converti. Les gaz peuvent ensuite passer sur une colonne absorbante réfrigérée pour condenser l'acrylonitrile, l'acétonitrile et l'acide cyanhydrique, les vapeurs non condensées contenant principalement du propane non converti, du propylène, des hydrocarbures légers et le cas échéant du CO₂. On peut ensuite séparer l'acrylonitrile et l'acide cyanhydrique de l'acétonitrile par distillation, puis distiller à son tour le mélange acrylonitrile-acide cyanhydrique récupéré pour séparer l'acrylonitrile de l'acide cyanhydrique.

Les exemples ci-après illustrent la présente invention.

### EXEMPLES DE PREPARATION D'OXYDES MIXTES.

### EXEMPLE 1 - Préparation de l'oxyde mixte (A) selon l'invention, de formule empirique suivante :V Sb5 Ti5 Ox

On prépare une solution de 10,92 g de TiCl₄ anhydre, de 3,05 g d'acétylacétonate de vanadyle et de 17,22 g de SbCl₅ dans 100 ml d'éthanol absolu.

La solution éthanolique est versée goutte à goutte dans 500 ml de solution aqueuse contenant de l'acétate d'ammonium (2 moles/litre) afin d'avoir un pH initial de l'ordre de 7,0. Pendant la précipitation des oxydes mixtes de V, Sb et Ti, le pH qui tend à diminuer en raison de la libération d'acide chlorhydrique, est maintenu constant par addition d'une solution concentrée d'ammoniaque (à 30 % en poids).

Le précipité ainsi formé est isolé par filtration, lavé à l'eau, séché pendant 12 h à 140 °C, puis il est calciné pendant 3 h à 700 °C.

Le composé de formule V Sb₅ Ti₅ Oₓ ainsi obtenu est ensuite comprimé sous une pression de 4300 bars. On obtient alors des pastilles de 3 cm de diamètre et d'environ 0,5 cm d'épaisseur. Ces pastilles sont concassées en éclats de granulométrie comprise entre 0,3 et 0,8 cm constituant le catalyseur (A) conforme à l'invention.

### EXEMPLES 2 ET 3 - Préparation de différents oxydes mixtes selon l'invention.

On prépare différents oxydes mixtes, en suivant le mode opératoire décrit dans l'exemple 1, mais en utilisant des quantités différentes d'acétylacétonate de vanadyle (exemple 3) ou de pentachlorure d'antimoine (exemple 2).

On obtient ainsi les oxydes mixtes selon l'invention présentant les formules suivantes :
- exemple 2: VSb₁₀Ti₅Oₓ (référence D)
- exemple 3: VSb₁₀Ti₁₀Oₓ (référence E)

### ESSAI COMPARATIF 1 - Préparation de l'oxyde mixte (B), préparé selon un procédé de l'art antérieur,de formule empirique suivante : V Sb5 Ti5 Ox

On prépare l'oxyde mixte de composition V Sb₅ Ti₅ Oₓ en opérant en 3 étapes par coprécipitation en milieu aqueux.

Dans 100 ml d'une solution H₂O/HCl de pH 0,5-1,0 ,on dissout 9,52g de tétrachlorure de titane. La solution est maintenue à basse température au moyen d'un bain glace/eau. Puis elle est réchauffée jusqu'à 90°C et maintenue à cette température pendant 1 heure.

Dans une deuxième solution analogue à la précédente, on dissout 2,65 g d'acétylacétonate de vanadyle et de 15,0g de pentachlorure d'antimoine. Cette solution est alors versée dans une solution tampon d'acétate d'ammonium dont le pH est maintenu constant à environ 7 par ajout d'ammoniaque.

Les deux solutions ainsi préparées sont mélangées et laissées sous agitation pendant 30 minutes. L'oxyde mixte qui précipite est séparé par centrifugation, lavé, séché à 140 °C pendant 12 heures, puis il est calciné pendant 3 heures à 700 °C.

Le composé de formule V Sb₅ Ti₅ Oₓ ainsi obtenu est ensuite comprimé sous une pression de 4300 bars. On obtient alors des pastilles de 3 cm de diamètre et d'environ 0,5 cm d'épaisseur. Ces pastilles sont concassées en éclats de granulométrie comprise entre 0,3 et 0,8 cm constituant le catalyseur (B) non conforme à l'invention.

### ESSAI COMPARATIF 2 - Préparation de l'oxyde mixte (J), préparé selon un procédé de l'art antérieur,de formule empirique suivante : V Sb5 Ti5 Ox

On prépare l'oxyde mixte de composition V Sb Ti_{6,75} Oₓ en opérant selon le mode opératoire décrit dans l'exemple M du brevet US 5 008 427.

Le composé de formule V Sb₅ Ti₅ Oₓ ainsi obtenu est ensuite comprimé sous une pression de 4300 bars. On obtient alors des pastilles de 3 cm de diamètre et d'environ 0,5 cm d'épaisseur. Ces pastilles sont concassées en éclats de granulométrie comprise entre 0,3 et 0,8 cm constituant le catalyseur (J) non conforme à l'invention.

### EXEMPLES 4 A 6 - Préparation de différents oxydes mixtes selon l'invention.

On prépare différents oxydes mixtes, en suivant le mode opératoire décrit dans l'exemple 1, mais en utilisant des quantités différentes d'acétylacétonate de vanadyle, de tétrachlorure de titane ou de pentachlorure d'antimoine.

On obtient ainsi les oxydes mixtes selon l'invention présentant les formules suivantes :
- exemple 4: VSbTi_{6,75}Oₓ (référence F)
- exemple 5: VSb_{1,5}Ti_{0,2}Oₓ (référence H)
- exemple 6: VSb_{1,5}Ti₁Oₓ (référence I)

### CARACTERISATION DES OXYDES MIXTES PREPARES

Les différents oxydes mixtes A, D, E, F, H et I selon l'invention et B non conforme à l'invention ont été caractérisés par diffraction des rayons X sur un diffractomètre de marque PHILIPS P1800 dans la gamme de mesure 10° à 70° en 2θ. Les raies caractéristiques des différents oxydes ont ensuite été comparées aux raies caractéristiques des produits purs reportés dans International Tables of X. Ray cristallography.

Les diffractogrammes obtenus mettent clairement en évidence que les catalyseurs (A), (D) et (E) sont constitués d'une phase rutile dont les paramètres de maille sont très proches de ceux de TiO₂ rutile. Le diffractogramme de l'oxyde (B) de l'art antérieur montre que l'oxyde (B) est constitué d'une phase TiO₂ rutile et d'oxyde Sb₆O₁₃.

### MODE OPERATOIRE GENERAL DES TESTS D'AMMOXYDATION

L'échantillon de catalyseur est préalablement porté sur un banc de mesure à la température de 150°C sous balayage d'hélium pendant 10 min, puis il est soumis à un flux gazeux dont la composition sera précisée pour chaque exemple et qui renferme du propane, de l'ammoniac, de l'oxygène, de l'hélium et le cas échéant de la vapeur d'eau.

La pression totale du mélange réactionnel comprise entre 1 et 6 bar, sera également précisée pour chaque exemple.

Le débit total gazeux est défini de façon à avoir une vitesse volumique horaire (WH) comprise entre 100 et 36000 h⁻¹, dont la valeur précise sera indiquée pour chaque exemple.

Volume de phase active : 25 cm³.

Le principe du test d'ammoxydation du propane est le suivant: >
- On porte le catalyseur à une température T₁, par exemple 310°C, et après 30 min de stabilisation à la température T₁, on détermine par chromatographie en phase gazeuse la composition du mélange en sortie de réacteur.
- On calcule les pourcentages de conversion et les sélectivités obtenus sur le catalyseur examiné à la température d'entrée T₁ par des relations du type : conversion du propane (en moles %) = propane transformé/propane introduit sélectivité en acrylonitrile (en moles %) = propane transformé en acrylonitrile/propane converti.
- On porte ensuite le catalyseur de 310 à 550°C par incrément de 20°C et on détermine toutes les 40 min les pourcentages de conversion et les sélectivités.

Dans les exemples d'ammoxydation ci-après, les conventions suivantes sont utilisées :
Temp = température de la réaction
TTC₃H₈ = conversion du propane
SACN = sélectivité en acrylonitrile
SACN+C₃H₆ = sélectivité en acrylonitrile et propylène
SAMMOX = sélectivité en acetonitrile, en acide cyanhydrique et en autres sous-produits d'ammoxydation
Prod ACN = productivité en acrylonitrile exprimée en g d'acrylonitrile formé/litre de catalyseur x heure.

Le complément à 100 % des sélectivités correspond à la formation de CO et de CO₂ ainsi qu'éventuellement de méthane, d'éthane et d'éthylène.

### EXEMPLES D'AMMOXYDATION DU PROPANE

### EXEMPLES 7 A 12 ET ESSAIS COMPARATIFS 3 ET 4

On réalise l'ammoxydation du propane, comme décrit précédemment, en utilisant comme catalyseurs les phases actives constituées par les oxydes mixtes de l'invention A, D, E, F, H et I et les oxydes mixtes non conformes à l'invention B et J.

Les conditions particulières mises en oeuvre sont les suivantes :
- vitesse volumique horaire = 1700 h⁻¹
- pression totale = 1,3 bar
- composition volumique du mélange réactionnel :
   C₃H₈ = 25 %
   NH₃ = 10 %
   O₂ = 20 %
   He = 45 %

Les conditions de température et les résultats obtenus sont rassemblés dans le tableau 1 ci-après.

**Tableau 1**

| Exemples | Catalyseur utilisé | Temp (°C) | TT C3H8 (en %) | SACN (en %) | SACN + C3H6 (en %) | SAMMOX (en %) | Prod ACN (g/lxh) |
|---|---|---|---|---|---|---|---|
| Exemple 7 | A | 388 | 6 | 25 | 31 | 42 | 15 |
| | | 408 | 11 | 34 | 39 | 28 | 37 |
| | | 428 | 22 | 36 | 39 | 14 | 79 |
| | | 447 | 24 | 43 | 46 | 15 | 102 |
| Exemple 8 | D | 404 | 12 | 40 | 48 | 23 | 48 |
| | | 422 | 14 | 46 | 51 | 21 | 71 |
| | | 442 | 16 | 53 | 56 | 16 | 83 |
| | | 461 | 18 | 53 | 57 | 15 | 95 |
| Exemple 9 | E | 407 | 13 | 32 | 38 | 25 | 41 |
| | | 426 | 19 | 39 | 43 | 21 | 74 |
| | | 445 | 19 | 49 | 52 | 14 | 92 |
| | | 465 | 19 | 51 | 54 | 14 | 96 |
| Exemple 10 | F | 407 | 8 | 21 | 48 | 33 | 17 |
| | | 427 | 13 | 25 | 40 | 36 | 32 |
| | | 447 | 17 | 31 | 42 | 23 | 52 |
| | | 468 | 21 | 36 | 43 | 18 | 75 |
| Exemple 11 | H | 389 | 10 | 29 | 46 | 25 | 29 |
| | | 407 | 14 | 32 | 46 | 23 | 44 |
| | | 428 | 21 | 35 | 47 | 18 | 73 |
| | | 446 | 24 | 37 | 50 | 17 | 88 |
| Exemple 12 | I | 388 | 15 | 20 | 35 | 30 | 30 |
| | | 408 | 20 | 35 | 45 | 29 | 69 |
| | | 425 | 24 | 37 | 44 | 12 | 88 |
| | | 446 | 25 | 39 | 48 | 7 | 97 |
| Essai comparatif 3 | B | 424 | 5 | 60 | 83 | 8 | 30 |
| | | 443 | 7 | 58 | 77 | 8 | 40 |
| | | 464 | 9 | 49 | 72 | 6 | 44 |
| | | 485 | 15 | 35 | 57 | 5 | 52 |
| Essai comparatif 4 | J | 426 | 2 | 22 | 57 | 22 | 4 |
| | | 448 | 3 | 32 | 63 | 21 | 9 |
| | | 468 | 4 | 42 | 67 | 16 | 17 |

## Revendications

1. Procédé de préparation d'un oxyde mixte comportant du vanadium, de l'antimoine et du titane, répondant à la formule empirique générale (I) :
V Sbₐ Ti_{b} Oₓ (I)
dans laquelle :
- a représente un nombre entier ou fractionnaire égal ou supérieur à 0,1
- b représente un nombre entier ou fractionnaire égal ou supérieur à 0,1
- x représente un nombre entier ou fractionnaire déterminé par le degré d'oxydation des autres éléments,
caractérisé en ce que :
- des composés respectifs de vanadium, d'antimoine et de titane sont mis en solution dans au moins un alcool saturé,
- la solution alcoolique ainsi obtenue est mise en contact avec de l'eau, afin de précipiter l'oxyde mixte,
- l'oxyde mixte obtenu est séparé et calciné.

2. Procédé selon la revendication 1, caractérisé en ce qu'il est appliqué à la préparation d'oxydes mixtes de formule (I) pour lesquels :
- a représente un nombre entier ou fractionnaire égal ou inférieur à 100 et de préférence un nombre entier ou fractionnaire de 0,5 à 50
- b représente un nombre entier ou fractionnaire égal ou inférieur à 100 et de préférence un nombre entier ou fractionnaire de 1 à 40
- x représente un nombre entier ou fractionnaire déterminé par le degré d'oxydation des autres éléments.

3. Procédé selon l'une des revendications 1 ou 2, caractérisé en ce que le composé soluble du vanadium est choisi parmi l'acétylacétonate de vanadyle, le trichlorure de vanadyle, le trifluorure de vanadium, le tétrafluorure de vanadium, le pentafluorure de vanadium, le tribromure de vanadium, le dichlorure de vanadium, le tdchlorure de vanadium, le tétrachlorure de vanadium, le triiodure de vanadium.

4. Procédé selon l'une des revendications 1 à 3, caractérisé en ce que le composé soluble de l'antimoine est choisi parmi le pentachlorure d'antimoine, le trichlorure d'antimoine, le tribromure d'antimoine, le trifluorure d'antimoine, le triiodure d'antimoine, le trioxyde d'antimoine, l'hydrure d'antimoine.

5. Procédé selon l'une des revendications 1 à 4, caractérisé en ce que le composé soluble du titane est choisi parmi le dichlorure de titane, le tétrachlorure de titane, le trichlorure de titane, le tribromure de titane, le tétrabromure de titane,.le tétrafluorure de titane, le diiodure de titane.

6. Procédé selon l'une des revendications 1 à 5, caractérisé en ce que les alcools saturés mis en oeuvre sont les alcanols et les cycloalcanols et de préférence les alcanols ayant de 1 à 6 atomes de carbone et le cyclohexanol.

7. Procédé selon l'une des revendications 1 à 6, caractérisé en ce que la solution alcoolique obtenue est ensuite mélangée à de l'eau, de manière à précipiter les oxydes mixtes, de préférence à une solution aqueuse d'un sel d'ammonium, tel qu'un carboxylate d'ammonium, l'oxalate d'ammonium, le carbonate d'ammonium, l'hydrogénophosphate d'ammonium, permettant d'avoir un pH compris entre 5 et 9 et de préférence proche de 7.

8. Procédé selon la revendication 7, caractérisé en ce que l'on maintient la valeur du pH de la solution à une valeur proche de 7, en neutralisant progressivement l'acidité formée lors de la précipitation des oxydes mixtes à l'aide d'un composé basique, de préférence par ajout progressif contrôlé d'ammoniaque.

9. Procédé selon l'une des revendications 1 à 8, caractérisé en ce que après séparation des oxydes mixtes, ceux-ci sont soumis à une calcination à une température de 400 °C à 800 °C, et de préférence de 500 °C à 750 °C.

10. Procédé d'ammoxydation d'alcanes en phase vapeur en présence d'un catalyseur solide comportant au moins une phase active, caractérisé en ce que la dite phase active comprend au moins un oxyde mixte préparé selon l'une des revendications 1 à 9, répondant à la formule générale empirique (I).

11. Procédé selon la revendication 10, caractérisé en ce que des alcanes ayant de 3 à 12 atomes de carbone par molécule, choisis de préférence parmi le propane et l'isobutane, sont mis à réagir en phase vapeur avec de l'ammoniac et de l'oxygène.

12. Procédé selon l'une des revendications 10 ou 11, caractérisé en ce que au sein du gaz réactif, constitué par l'alcane, l'ammoniac, l'oxygène, la teneur en alcane est de préférence comprise entre 5 et 70 %, la teneur en ammoniac est de préférence comprise entre 3 et 50 % et la teneur en oxygène est de préférence comprise entre 3 et 45 %.

13. Procédé selon l'une des revendications 10 à 12, caractérisé en ce que la température de réaction est comprise entre 300°C et 550°C et, de préférence, entre 400°C et 500°C.

## Claims

1. Process for the preparation of a mixed oxide comprising vanadium, antimony and titanium, corresponding to the general empirical formula (I):
VSbₐTi_{b}Oₓ (I)
in which:
- a represents an integral or fractional number equal to or greater than 0.1
- b represents an integral or fractional number equal to or greater than 0.1
- x represents an integral or fractional number determined by the oxidation state of the other elements,
characterized in that:
- respective compounds of vanadium, of antimony and of titanium are dissolved in at least one saturated alcohol,
- the alcoholic solution thus obtained is brought into contact with water in order to precipitate the mixed oxide, and
- the mixed oxide obtained is separated off and calcined.

2. Process according to claim 1, characterized in that it is applied to the preparation of mixed oxides of formula (I) for which:
- a represents an integral or fractional number equal to or less than 100, and preferably an integral or fractional number from 0.5 to 50
- b represents an integral or fractional number equal to or less than 100, and preferably an integral or fractional number from 1 to 40
- x represents an integral or fractional number determined by the oxidation state of the other elements.

3. Process according to either of claims 1 and 2, characterized in that the soluble vanadium compound is selected from vanadyl acetylacetonate, vanadyl trichloride, vanadium trifluoride, vanadium tetrafluoride, vanadium pentafluoride, vanadium tribromide, vanadium dichloride, vanadium trichloride, vanadium tetrachloride and vanadium triiodide.

4. Process according to one of claims 1 to 3, characterized in that the soluble antimony compound is selected from antimony pentachloride, antimony trichloride, antimony tribromide, antimony trifluoride, antimony triiodide, antimony trioxide and antimony hydride.

5. Process according to one of claims 1 to 4, characterized in that the soluble titanium compound is selected from titanium dichloride, titanium tetrachloride, titanium trichloride, titanium tribromide, titanium tetrabromide, titanium tetrafluoride and titanium diiodide.

6. Process according to one of claims 1 to 5, characterized in that the saturated alcohols employed are alkanols and cycloalkanols, and preferably alkanols having 1 to 6 carbon atoms and cyclohexanol.

7. Process according to one of claims 1 to 6, characterized in that the alcoholic solution obtained is subsequently mixed with water so as to precipitate the mixed oxides, preferably with an aqueous solution of an ammonium salt such as ammonium carboxylate, ammonium oxalate, ammonium carbonate or ammonium hydrogen phosphate, which provides a pH of between 5 and 9 and preferably of close to 7.

8. Process according to claim 7, characterized in that the pH of the solution is maintained at a value close to 7 by progressively neutralizing the acidity which is formed during the precipitation of the mixed oxides by means of a basic compound, preferably by controlled, progressive addition of aqueous ammonia.

9. Process according to one of claims 1 to 8, characterized in that, after the mixed oxides have been separated off, they are calcined at a temperature of from 400°C to 800°C, and preferably from 500°C to 750°C.

10. Process for the ammoxidation of alkanes in the vapour phase in the presence of a solid catalyst containing at least one active phase, characterized in that the said active phase comprises at least one mixed oxide prepared according to one of claims 1 to 9 and corresponding to the general empirical formula (I).

11. Process according to claim 10, characterized in that alkanes having from 3 to 12 carbon atoms per molecule, selected preferably from propane and isobutane, are reacted in the vapour phase with ammonia and oxygen.

12. Process according to either of claims 10 and 11, characterized in that, within the reactive gas composed of alkane, ammonia and oxygen, the alkane content is preferably between 5 and 70 %, the ammonia content is preferably between 3 and 50 % and the oxygen content is preferably between 3 and 45 %.

13. Process according to one of claims 10 to 12, characterized in that the reaction temperature is between 300°C and 550°C, and preferably between 400°C and 500°C.

## Patentansprüche

1. Verfahren zur Herstellung eines gemischten Oxids, umfassend Vanadium, Antimon und Titan, das der allgemeinen empirischen Formel (I)
V Sbₐ Ti_{b} Oₓ (I)
entspricht, in der
- a eine ganze oder gebrochene Zahl von gleich oder höher 0,1 darstellt,
- b eine ganze oder gebrochene Zahl von gleich oder höher 0,1 darstellt,
- x eine ganze oder gebrochene Zahl darstellt, die durch den Oxidationsgrad der anderen Elemente bestimmt ist,
dadurch gekennzeichnet, daß
- die jeweiligen Verbindungen von Vanadium, Antimon und Titan in mindestens einem gesättigten Alkohol in Lösung gebracht werden,
- die auf diese Weise erhaltene alkoholische Lösung in Kontakt mit Wasser gebracht wird, um das gemischte Oxid auszufällen, und
- das erhaltene gemischte Oxid abgetrennt und kalziniert wird.

2. Verfahren nach Anspruch 1, dadurch gekennzeichnet, daß es bei der Herstellung von gemischten Oxiden der Formel (I) angewendet wird, in der
- a eine ganze oder gebrochene Zahl von gleich oder niedriger 100 und vorzugsweise eine ganze oder gebrochene Zahl von 0,5 bis 50 darstellt,
- b eine ganze oder gebrochene Zahl von gleich oder niedriger 100 und vorzugsweise eine ganze oder gebrochene Zahl von 1 bis 40 darstellt,
- x eine ganze oder gebrochene Zahl darstellt, die durch den Oxidationsgrad der anderen Elemente bestimmt ist.

3. Verfahren nach einem der Ansprüche 1 oder 2, dadurch gekennzeichnet, daß die lösliche Verbindung des Vanadiums ausgewählt wird unter Vanadyl-acetylacetonat, Vanadyl-trichlorid, Vanadium-trifluorid, Vanadium-tetrafluorid, Vanadium-pentafluorid, Vanadium-tribromid, Vanadium-dichlorid, Vanadium-trichlorid, Vanadium-tetrachlorid, Vanadium-triiodid.

4. Verfahren nach einem der Ansprüche 1 bis 3, dadurch gekennzeichnet, daß die lösliche Verbindung des Antimons ausgewählt wird unter Antimon-pentachlorid, Antimon-trichlorid, Antimon-tribromid, Antimon-trifluorid, Antimon-triiodid, Antimon-trioxid, Antimon-hydrid.

5. Verfahren nach einem der Ansprüche 1 bis 4, dadurch gekennzeichnet, daß die lösliche Verbindung des Titans ausgewählt wird unter Titan-dichlorid, Titan-tetrachlorid, Titan-trichlorid, Titan-tribromid, Titan-tetrabromid, Titan-tetrafluorid, Titan-diiodid.

6. Verfahren nach einem der Ansprüche 1 bis 5, dadurch gekennzeichnet, daß die eingesetzten gesättigten Alkohole Alkanole und Cycloalkanole und vorzugsweise die Alkanole mit 1 bis 6 Kohlenstoffatomen und Cyclohexanol sind.

7. Verfahren nach einem der Ansprüche 1 bis 6, dadurch gekennzeichnet, daß die erhaltene alkoholische Lösung anschließend mit Wasser vermischt wird, um die gemischten Oxide auszufällen, vorzugsweise mit einer wäßrigen Lösung eines Ammoniumsalzes wie einem Ammoniumcarboxylat, Ammoniumoxalat, Ammoniumcarbonat, Ammoniumhydrogenphosphat, um einen pH-Wert zwischen 5 und 9 zu ermöglichen und vorzugsweise in der Nähe von 7.

8. Verfahren nach Anspruch 7, dadurch gekennzeichnet, daß man den pH-Wert der Lösung auf einem Wert von etwa 7 hält, indem man nach und nach die bei der Fällung der gemischten Oxide gebildete Azidität mit Hilfe einer basischen Verbindung neutralisiert, vorzugsweise durch schrittweise und kontrollierte Zugabe von Ammoniak.

9. Verfahren nach einem der Ansprüche 1 bis 8, dadurch gekennzeichnet, daß die gemischten Oxide nach ihrer Abtrennung einer Kalzinierung bei einer Temperatur von 400 °C bis 800 °C, vorzugsweise von 500 °C bis 750 °C, unterzogen werden.

10. Verfahren zur Ammoxidation von Alkanen in der Dampfphase in Anwesenheit eines festen Katalysators, der mindestens eine aktive Phase umfaßt, dadurch gekennzeichnet, daß die genannte aktive Phase mindestens ein gemischtes Oxid umfaßt, hergestellt nach irgendeinem der Ansprüche 1 bis 9 und entsprechend der allgemeinen empirischen Formel (I).

11. Verfahren nach Anspruch 10, dadurch gekennzeichnet, daß die Alkane, die 3 bis 12 Kohlenstoffatome pro Molekül besitzen, vorzugsweise ausgewählt werden unter Propan und Isobutan, die in der Dampfphase mit Ammoniak und Sauerstoff zur Reaktion gebracht werden.

12. Verfahren nach einem der Ansprüche 10 oder 11, dadurch gekennzeichnet, daß in dem reaktiven Gas, das aus dem Alkan, Ammoniak und Sauerstoff besteht, der Gehalt an Alkan vorzugsweise zwischen 5 % und 70 %, der Gehalt an Ammoniak vorzugsweise zwischen 3 % und 50 % und der Gehalt an Sauerstoff vorzugsweise zwischen 3 % und 45 % betragen.

13. Verfahren nach einem der Ansprüche 10 bis 12, dadurch gekennzeichnet, daß die Temperatur der Reaktion zwischen 300 °C und 550 °C, vorzugsweise zwischen 400 °C und 500 °C liegt.
